(19)

Europäisches
Patentamt
European
Patent Office
Office européen
des brevets

(11) **EP 1 961 442 A1**

(12) **EUROPEAN PATENT APPLICATION**

(43) Date of publication:
**27.08.2008 Bulletin 2008/35**

(51) Int Cl.:
*A61M 27/00* *(2006.01)*

(21) Application number: **08009084.8**

(22) Date of filing: **29.06.2005**

(84) Designated Contracting States:
**AT BE BG CH CY CZ DE DK EE ES FI FR GB GR
HU IE IS IT LI LT LU MC NL PL PT RO SE SI SK TR**

(30) Priority: **30.06.2004 US 881497**

(62) Document number(s) of the earlier application(s) in
accordance with Art. 76 EPC:
**05254055.6 / 1 614 442**

(71) Applicant: **Codman & Shurtleff, Inc.
Raynham,
Massachusetts 02767 (US)**

(72) Inventors:
• **Mauge, Christophe
  Melrose, MA 02176 (US)**
• **Dimauro, Thomas
  Southboro, MA 01772 (US)**

• **Attawia, Mohamed
  Canton, MA 02021 (US)**
• **Sehran, Hassan
  Easton, MA 02975 (US)**
• **Sutton, Jeffrey
  Medway, MA 02053 (US)**
• **Beardsley, Timothy
  Kingston, MA 02364 (US)**

(74) Representative: **Mercer, Christopher Paul et al
Carpmaels & Ransford
43, Bloomsbury Square
London WC1A 2RA (GB)**

Remarks:
This application was filed on 16-05-2008 as a
divisional application to the application mentioned
under INID code 62.

(54) **Hydrocephalus shunt**

(57)    A hydrocephalus shunt has photocatalytic capabilities. The shunt is used to treat hydrocephalus by inserting into a human cranium a hydrocephalus shunt having a component having a surface. Thereafter, a reactive oxygen species is produced on the component surface. The component may be a catheter. The shunt may also include a light source (453). The shunt may alternatively include a light port (61,455).

# FIG. 6

EP 1 961 442 A1

**Description**

**BACKGROUND OF THE INVENTION**

Hydrocephalus is a condition afflicting patients who are unable to regulate cerebrospinal fluid flow through their body's own natural pathways. Produced by the ventricular system, cerebrospinal fluid (CSF) is normally absorbed by the body's venous system. In a patient suffering from hydrocephalus, the cerebrospinal fluid is not absorbed in this manner, but instead accumulates in the ventricles of the patient's brain. If left untreated, the increasing volume of fluid elevates the patient's intracranial pressure and can lead to serious medical conditions such as subdural hematoma, compression of the brain tissue, and impaired blood flow.

The treatment of hydrocephalus has conventionally involved draining the excess fluid away from the ventricles and rerouting the cerebrospinal fluid to another area of the patient's body, such as the abdomen or vascular system. A drainage system, commonly referred to as a shunt, is often used to carry out the transfer of fluid. In order to install the shunt, typically a scalp incision is made and a small hole is drilled in the skull. A proximal, or ventricular, catheter is installed in the ventricular cavity of the patient's brain, while a distal, or drainage, catheter is installed in that portion of the patient's body where - the excess fluid is to be reintroduced. To regulate the flow of cerebrospinal fluid and maintain the proper pressure in the ventricles, a pump or one-way control valve can be placed between the proximal and distal catheters. Such valves can comprise a ball-in-cone mechanism as illustrated and described in U.S. Patent Nos. 3,886,948, 4,332,255, 4,387,715, 4,551,128, 4,595,390, 4,615,691, 4,772,257, and 5,928,182, all of which are hereby incorporated by reference. When properly functioning, these shunt systems provide an effective manner of regulating CSF in hydrocephalus patients.

After implantation and use over extended periods of time, these shunt systems tend to malfunction due to shunt occlusion. Frequently, the blockage occurs within the ventricular catheter. The obstruction can result from a number of problems, such as clotting, bloody CSF, excess protein content in the CSF, inflammatory or ependymal cells, brain debris, infection, or by choroid plexus or brain parenchyma in-growth through the openings of the ventricular catheter. Another potential cause of ventricular catheter occlusion is a condition known as slit ventricle syndrome in which the ventricular cavity collapses, thus blocking the openings of the ventricular catheter. If left untreated, the occlusion of the ventricular catheter can slow down and even prevent the ability of the shunt valve to refill, thereby rendering the shunt system ineffective.

In the past, the remedy for a clogged proximal catheter was to surgically remove and replace the catheter, which involved a risk of damage to the brain tissue or hemorrhage. The current trend is to rehabilitate the catheter in place through less invasive means. This can be accomplished in a procedure generally known as shunt or ventricular catheter revision which involves reaming the clogged catheter in its implanted state until the blockage is removed to thereby reestablish CSF flow through the ventricular catheter. Many shunt valves, such as the ones described in U.S. Patent Nos. 4,816,016 and 5,176,627, are provided with a domed silicone reservoir that enables access to the attached ventricular catheter so that the system can be flushed out for this very reason. The self sealing silicone dome can be pierced with a small needle to gain entry to the attached catheter, without affecting the ability of the dome to re-seal after the needle has been withdrawn. In some domed valves with right angle access, i.e., where the ventricular catheter extends at a 90 degree angle to the drainage catheter, a surgeon can gain entry to the clogged ventricular catheter percutaneously by inserting a rigid endoscopic instrument such as an endoscopic cutting tool or endoscopic electrode through the dome of the valve and straight down to the attached catheter. Thereafter, the obstruction can be cleared by cutting, cauterizing, or coagulating using the endoscopic instrument.

In addition, infection is a well known complication associated with hydrocephalus shunts. It is well known that infections occur in about 5% to about 10% of all hydrocephalus shunt implantations. It is believed that a majority of these infections occur via transmission from microbes upon the surgical gloves, the patient's skin, implants or instruments. Unlike routine systemic infections, infections associated with implants ("periprosthetic infections") are particularly troublesome.

First, it has been reported that certain biomaterials cause an abnormal and inferior immune response. In short, a portion of the immune response is provided by the release of superoxide ions, such as hydroxyl radicals, that are lethal to microbes. However, when a periprosthetic infection occurs, it has been reported that certain biomaterials cause abnormal neutrophil activity, resulting in an inferior non-productive immune response. Shanbhag, J. Biomed. Mar. Res., Vol. 26, 185-95, 1992.

Second, it appears that the presence of the implant surface helps the microbes survive both the immune response and antibiotic treatment. In particular, microbes of concern attach to the implant surface and form a polymer-like glaze (or "biofilm") between themselves and the local environment. This biofilm acts as an effective barrier to both neutrophils and antibiotics.

Therefore, it is an object of the present invention to provide a hydrocephalus shunt adapted to prevent and /or treat occlusions and infections.

## SUMMARY OF THE INVENTION

The present inventors have developed a novel hydrocephalus shunt capable of producing reactive oxygen species (ROS). The reactive oxygen species (ROS) produced by this shunt are potent oxidizing agents capable of therapeutically treating the shunt and its local environment. However, because of the potency of the ROS, the ROS typically react very quickly with surrounding organic material and so have only a very local effect (e.g., 5-20 nm). Accordingly, the ROS treatment is very safe.

In some embodiments, the ROS produced from the shunt kill microbes present in the vicinity of the shunt, thereby preventing or treating infection. In some embodiments, the ROS produced by the shunt can oxidize a biofilm produced by microbes and attached to a surface of the shunt. In some embodiments, the ROS produced by the shunt oxidize organic matter forming an occlusion in a shunt lumen.

Therefore, in accordance with the present invention, there is provided a method of treating hydrocephalus that includes: inserting into a human cranium a hydrocephalus shunt having a component having a surface, and producing reactive oxygen species on the component surface.

In preferred embodiments, the hydrocephalus shunt has a photocatalytic material. Upon illumination with a predetermined wavelength of light and in the presence of water, the photocatalytic material locally generates the desired reactive oxygen species. In some embodiments, the photocatalytic material is provided as a layer upon a surface of a catheter base material. Preferably, the photocatalytic layer comprises a semiconductor material, and is preferably a metal oxide, more preferably titanium oxide. In other embodiments, the photocatalytic material is compounded into the catheter base material. Titanium dioxide has been shown to have photocatalytic activity for generating ROS.

In some embodiments, the shunt is illuminated with an external light source. In some embodiments, a fiber-optic cable is connected to the external light source and passed through the patient's skin to connect with the implanted shunt. Alternatively, the light can pass through the skin as transcutaneous (red) light, or can be an internal source such as an LED.

In some embodiments, the photocatalytic layer is doped to enhance or prolong the photocatalytic effect. Some such dopants include, but are not limited to, metal alloys or ions of chromium and/or vanadium; phosphorescent compounds, ligands, or ions; organic compounds containing oxygen-rich chemical species such as peroxides, superoxides, acids, esters, ketones, aldehydes, ethers, epoxides, and lactones; and organic compounds containing conjugated systems, such as photostabilizers and dyes. In preferred embodiments, the dopant allows the photocatalyst to produce ROS using longer wavelength light.

In some embodiments, the shunt is illuminated after implantation and all surgical manipulations have been performed. Such post-implantation illumination could be performed just prior to surgical closure, or after some period of time has elapsed by a percutaneous access approach using a fiber-optic delivery cable, or by a transcutaneous light source, or from an internal LED.

In some embodiments, a light port is incorporated into the shunt to provide efficient delivery and coupling of the light source energy to the photocatalytic layer. The light port could include a self-sealing gland, such as a self-sealing silicone domed reservoir, to prevent contamination and occlusion of the optical surface, thereby providing efficient energy transfer. Additionally, the light port could include a radiopaque marker, e.g. a tapered cylinder or other geometry, to allow the surgeon to efficiently direct a percutaneous needle with a fiber optic to the desired site under fluoroscopic guidance.

In some embodiments, the light port is provided on the device suitable to receive a hypodermic needle carrying a fiber optic and thereby deliver the light percutaneously to a light-receiving "reservoir" on the device. The reservoir is adapted to direct the light via a waveguide, optical fiber, or light pipe residing within the device to the photocatalytically functional areas.

The shunt device could further incorporate a waveguide layer to deliver light energy to the photocatalytic layer. The waveguide layer is preferably provided as longitudinal elements disposed within a catheter. It may be desirable for the waveguide layer to be of a different material than the photocatalytic layer to allow efficient energy transfer. For example, an undoped titanium oxide photocatalytic layer having a band gap energy requires light having a wavelength of less than 380 nm to be used to induce the photocatalytic effect. However, titanium oxide is moderately to strongly absorbing at wavelengths below ~ 450 nm and so would not function efficiently as a waveguide to propagate the light to all areas of the device. Accordingly, the use of a UV transmissive material such as silicon oxide, aluminum oxide, or other material with low absorption at the relevant wavelengths as the waveguide layer would allow the light to reach regions distant from the light port or entry point. In some embodiments, the waveguide is a polymer selected from the group consisting of silicones, urethanes, acrylics and polycarbonates.

In some embodiments, an at least partially reflecting layer is provided on the outer surfaces of a catheter to enhance the transmission of light energy down the catheter. In some preferred embodiments, silver metal is used as the reflective layer, having known desirable optically reflective properties as well as known anti-microbial properties. Alternative reflective materials include aluminum or gold metal.

In some embodiments, the use of dopants as described above, and particularly metal ions, modify the band gap energy of the titanium oxide layer such that visible light greater than 380 nm can be used to effectively induce the photocatalytic activity. In this system, the photocatalytic layer could also act as the waveguide layer, and the use of a partially reflective silver coating would enhance the internal reflection of the light to efficiently spread the light energy throughout the layer. The selection of silver also provides additional anti-microbial activity.

In some embodiments, at least one catheter of the shunt comprises more than one lumen, and is preferably either a dual lumen or triple lumen. In these embodiments, the secondary lumen accommodates an optical fiber adapted to deliver the light to at least a portion of the catheter. Preferably, the optical fiber is built into the shunt design during manufacture. After insertion and trimming of the end of the catheter in the valve area of the shunt, the trimmed end of the fiber optic cable is aligned with the light port and light reservoir by a mating fitting in order to receive light therefrom. In other embodiments, the mating fitting is adapted to deliver 360 degrees of light into the tubing cross-section (i.e., not just into the end of the fiber optic cable). Also, one may use a percutaneous fiber optic delivered into a secondary lumen, with a end of the fiber optic modified to deliver a portion of light perpendicular to the axis of the lumen.

## DESCRIPTION OF THE FIGURES

FIG. 1a is an exploded view of a hydrocephalus shunt of the present invention.

FIG. 1b is a cross-section of the ventricular catheter of FIG. 1a.

FIG. 2 depicts the treatment of an obstructed catheter.

FIGS. 3-5 and 7-15E are each cross-sections of portions of various ventricular catheters comprising photocatalytic materials.

FIG 6. is a schematic of a shunt of the present invention having an LED and internal antnenna.

FIG. 16 is a cross-section of a surface portion of a titanium implant having an oxidized surface, wherein the surface has been further bombarded with a dopant.

FIG. 17 is a cross-section of a portion of an implant having an intermediate waveguide layer and an upper photocatalytic layer.

FIG. 18A is a cross-section of a portion of an implant having an composite coating comprising a waveguide and a photocatalytic material.

FIG. 18B is a cross-section of a portion of an implant having an composite comprising a waveguide and a photocatalytic material dispersed within a base material of the implant.

FIG. 19 is a cross section of a needle containing a fiber optic cable.

FIG. 20 is a cross section of a portion of an implant having a port for connecting a fiber optic.

FIG. 21 is a cross-section of a portion of an implant having a waveguide layer, a photocatalytic layer, and an outer reflective layer.

FIG. 22 is a preferred implant of the present invention having a lower waveguide layer, an intermediate reflective layer, and an outer porous photocatalytic layer.

FIG. 23 is an implanted photocatalytic shunt powered by telemetry.

FIG. 24 is a schematic of a shunt of the present invention.

FIG. 25 is an embodiment of a valved shunt of the present invention embedded underneath the skin.

FIG. 26 is an embodiment of a preferred hydrocephalus shunt of the present invention embedded underneath the skin.

FIG.27 is a cross-section of a surface portion of a titanium component of a shunt, wherein the surface has been oxidized to produce a thick titania layer.

## DETAILED DESCRIPTION OF THE INVENTION

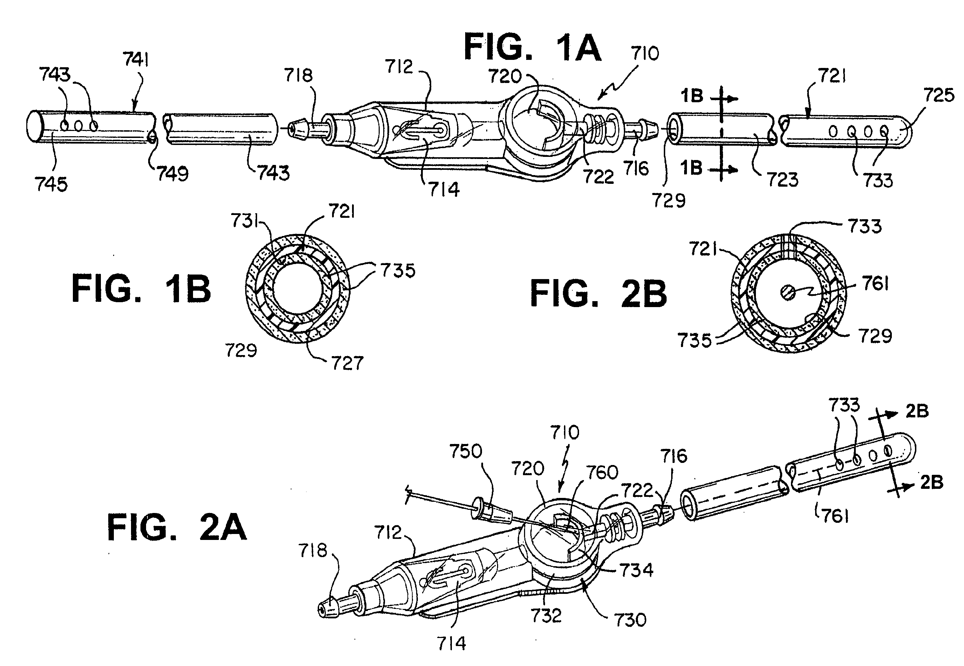

For the purposes of the present invention, "titanium dioxide" is also referred to as titania and $TiO_2$. A "UV light source" includes any light source emitting light having a maximum energy wavelength of between about 0.1 nm and about 380 nm. A "UVC light source" includes any light source emitting light having a maximum energy wavelength of between about 0.1 nm and less than 290 nm. A "UVB light source" includes any light source emitting light having a maximum energy wavelength of between 290 nm and less than 320 nm. A "UVA light source" includes any light source emitting light having a maximum energy wavelength of between 320 nm and less than 380 nm. A "visible light source" includes any light source emitting light having a maximum energy wavelength of between 380 nm and less than 780 nm. A "infrared light source" includes any light source emitting light having a maximum energy wavelength of between 780 nm and less than one million nm. A "reactive oxygen species" includes hydrogen peroxide, hydroxyl radicals, superoxide ion, and singlet oxygen and is also referred to as "ROS". For the purposes of the present invention, "silcone" refers to poly(dimethylsiloxane) material or PDS; the "ventricular catheter" may also be termed the "proximal catheter"; the "drainage catheter" may also be termed the "distal catheter".
The following US Patent Applications are incorporated by reference herein in their entirety: 10/774,105 filed on February 6, 2004 and entitled Implant Having a Photocatalytic Unit and 10/459406, filed June 11, 2003 and entitled Needle Guard Having Inherent Probe Directing Features (now published as US 2004-0254522).
Referring now to FIGS. 1a and 1b, there is provided a hydrocephalus shunt 710 for draining fluid within a patient, comprising:

> a) a housing 712 having a valve mechanism 714 therein for regulating fluid flow into and out of the shunt device, an inlet port 716 configured to receive a ventricular catheter 717, an outlet port 718 configured to receive a drainage catheter 719;
> b) ventricular catheter 721 attached to the inlet port;
> c) drainage catheter 731 attached to the outlet port.

Ventricular catheter 721 comprises a proximal end portion 723, a distal end portion 725 , an outer surface 727, and a longitudinal lumen 729 providing fluid connection with the housing and defining inner surface 731. A plurality of inlet holes 733 are provided in the proximal end portion of the ventricular catheter, providing fluid communication between the outer surface of the catheter and the catheter lumen. These holes are adapted to allow excess CSF present in the brain to drain into the shunt. Disposed upon the outer and inner surfaces of the ventricular catheter is a layer of a photocatalytic material 735 (as shown in FIG. 1b only).
Drainage catheter 741 comprises a proximal end portion 743, a distal end portion 745 , an outer surface 747, and a longitudinal lumen 749 providing fluid connection with the housing and defining inner surface 751. A plurality of outlet holes 733 are provided in the distal end portion of the drainage catheter, providing fluid communication between the outer surface of the drainage catheter and the drainage catheter lumen. These holes are adapted to allow CSF within the shunt to drain into another portion of the patient's body, such as into the heart or peritoneum. Disposed upon the outer and inner surfaces of the drainage catheter is a layer of a photocatalytic material (not shown).
The valve mechanism 714 can comprise any typical valve mechanism, such as the ball-in-cone valve illustrated and as described in U.S. Patent Nos. 3,886,948, 4,332,255, 4,387,715, 4,551,128, 4,595,390, 4,615,691, 4,772,257, and 5,928,182, all of which are hereby incorporated by reference. Of course, it is understood that the valve mechanism 14 can also comprise other suitable valves including programmable valves for controlling fluid flow in a shunt device as are known in the art.
In inlet port 716 is provided for attachment to a ventricular catheter that is to be implanted in a ventricular cavity of a hydrocephalus patient.
The outlet port 718 is configured to attach to a drainage catheter which would be placed in the region of the patient such as the peritoneal cavity where excess cerebrospinal fluid is to be reintroduced.
Also included with the shunt device 710 is a domed reservoir 720 that is in fluid communication with the inlet port 716 by way of channel 722. The domed reservoir 720 can be formed from a self-sealing silicone as is well known in the art, thereby enabling a needle to puncture the silicone dome for access to the shunt device 710 while still providing a seal to form upon withdrawal of the needle from the reservoir 720. The shunt device 10 has an in-line configuration, i.e., the inlet and outlet ports 716, 718 extend at an angle of about 180° with respect to one another. Preferred embodiments of the domed reservoir include those in U.S. Patent Publication 2004-0254522, filed June 11, 2003 and entitled Needle Guard Having Inherent Probe Directing Features, the specification of which is incorporated by reference in its entirety.
Now referring to FIG. 2, when the inlet holes of the shunt of FIG.1a become obstructed, the physician inserts a needle 750 having a fiber optic cable 760 into the domed reservoir and accesses the obstructed catheter. The physician then advances the fiber optic cable through the catheter until its UV light-emitting distal end 761 is in the vicinity of the obstructed inlet hole. Next, the physician activates the light source (not shown) associated with the fiber optic and illuminates the distal end of the catheter. In this particular example, the proximal catheter is made of silicone, while the photocatalytic layer is made of titania. Because both the silicone catheter and the titania are relatively transmissive to UV light, illumination of the distal end of the catheter effectively back lights the photocatalytic layer, thereby producing holes and electrons in the titania. These holes and electrons both oxidize and reduce water to produce ROS. These ROS then oxidize the organic matter causing the obstruction, thereby therapeutically treating the obstructed catheter.

It is believed that the ROS produced by the photocatalytic reaction of the present invention are produced in sufficient quantities to reduce the mass of protein matter causing an obstruction within the shunt.

Reports upon testing the ability of photocatalytic oxidation to oxidize bovine serum albumin, reported that about 80% of the mass of bovine serum albumin present upon the photocatalytic surface was oxidized within about 36 hours.

Since it is known that the proximal-most inlet hole of a ventricular catheter is most prone to blockage, in some embodiments, at least one of the catheter surfaces surrounding the proximal-most hole are coated with a photocatalytic layer. In some embodiments, at least the annular surface defined by the proximal-most inlet hole is coated with a photocatalytic layer.

It is further believed that the ROS produced by the photocatalytic reaction of the present invention are produced in sufficient quantities to kill neighboring macrophages that have called as part of the immune reaction to shunt implantation. As noted above, investigators have reported that products of inflammation are believed to be a major source of protein matter causing an obstruction within the shunt.

Without wishing to be tied to a theory, it is believed that the ROS generated by the PCO will have a cytotoxic effect upon macrophages present in the region surrounding to shunt. However, because of the high reactivity of the ROS, the cytotoxic effect will be localized only to the inflamed tissue in the 5-20 nm region around the shunt.

The beneficial use of ROS to mediate inflammation has been reported in the literature. It has been reported by Kereiakes, Circulation, 2003, 108:1310-5, that the generation of an ROS (in this case, singlet oxygen) in the vicinity of a cardiovascular stent mediates macrophage apoptosis: "Mononuclear cell inflammatory infiltrate in conjunction with monocyte colony stimulating factor has been implicated in the pathogenesis of vascular smooth muscle cell apoptosis, depletion and subsequent weakening of plaque infrastructural integrity, which precipitates plaque rupture." Likewise, according to Chou, Catheterization and Cardiovascular Interventions 57: 387-94 (2002), studies have shown that cellular damage to the atheromatous intima and media can be achieved by light exposure, but with the preservation of the elastic lamina and normal collagen of the adventitia, thereby suggesting that deep vessel structures may remain unchanged. The absence of mural inflammation, despite extensive cell death, is consistent with the regression of atherosclerotic plaque through apoptosis.

Without wishing to be tied to a theory, it is believed that the photocatalytic unit of the present invention works to effectively fight a periprosthetic infection (PPI) in the following manner:

It is known that neutrophils play a critical role in fighting infection in the body. It is believed that when the body recognizes a foreign body, such as an implant, signaling from the immune system calls neutrophils to the implant location. The neutrophils proceed to emit a number of infection-fighting molecules, including reactive oxygen species (ROS), such as superoxide ion. Without wishing to be tied to a theory, it is believed that the ROS, and the superoxide ion in particular, cause the death of the pathogenic bacteria by penetrating the cells wall of the bacteria.

Kaplan et al., J. Biomed. Mat. Res., 26, 1039-51 (1992) investigated the role played by neutrophils in periprosthetic infection (PPI) and found that the neutrophils prematurely emit their infection-fighting compounds and, when the infection is sustained, appear to exhaust their capability of manufacturing more of these infection fighting compounds. Accordingly, it appears that the body response to PPI include a dose of apparently potent compounds, but that dose is not sustained. When the release period ends, the body does not adequately respond to the PPI.

In sum, the typical immune response of the body to an infection involves the release of superoxide ions by local neutrophils in amounts that are lethal to the local bacteria, and that periprosthetic infection often arises due to the implant's interference with this natural activity.

When the semiconductor element of the PCO of the present invention is properly irradiated by the UV light source, it is believed that reactive oxygen species (ROS) are produced at the semiconductor surface and enter the body fluid adjacent the photocatalytic surface. These ROS include hydroxyl radicals (OH), hydrogen peroxide ($H_2O_2$), superoxide ion ($^-O_2$) and singlet oxygen (O) and appear to be the same ROS naturally produced by neutrophils in the natural immune response to PPI. However, whereas the neutrophil response is limited both in magnitude and duration, the PCO unit of the present invention can be tuned to emit ROS in both a magnitude and for a duration deemed appropriate for the extent of infection diagnosed by the clinician.

When an effective amount of light irradiates the photocatalytic surface of the prosthetic device of the present invention, the sensitized surface can effectively catalyze both the oxidation of water (to produce hydroxyl radicals OH) and the reduction of oxygen (to produce superoxide radicals $^-O_2$). Without wishing to be tied to a theory, it is believed that PCO may also produce significant amounts of hydrogen peroxide.

Accordingly, activation of the PCO unit disposed on the implant effectively produces and releases the same molecular units naturally released by the patient's full-strength immune system. Therefore, it is believed that at least the superoxide radicals $O_2$-produced by the PCO unit effectively kill at least the free floating bacteria that are not protected by a biofilm.

As stated above, it is believed that the PCO unit of the present invention causes the production of hydrogen peroxide near or upon the semiconductor surface. It is well known that hydrogen peroxide is lethal to bacteria. In some embodiments, the PCO unit produces a local concentration of hydrogen peroxide believed to be sufficient to kill Staphylococcus epidermis. In some embodiments, the PCO unit produces a local concentration of hydrogen peroxide in the range typically produced by natural neutrophils in response to an infection. In some embodiments, the PCO unit produces a local concentration of hydrogen peroxide believed to be sufficient to oxidize a biofilm.

As stated above, the PCO unit of the present invention causes the production of superoxide ion upon the semiconductor surface. It is well known that superoxide ion is lethal to bacteria. In some embodiments, the PCO unit produces a local concentration of superoxide ion believed to be sufficient to kill Staphylococcus epidermis. In some embodiments, the PCO unit produces a local concentration of superoxide ion in the range typically produced by natural neutrophils in response to an infection. In some embodiments, the PCO unit produces a local concentration of superoxide ion believed to be sufficient to oxidize a biofilm.

As stated above, the PCO unit of the present invention causes the production of hydroxyl radicals upon the semiconductor surface. It is well known that hydroxyl radicals are particularly lethal to bacteria. In some embodiments, the PCO unit produces a local concentration of hydroxyl radicals believed to be sufficient to kill Staphylococcus epidermis. In some embodiments, the PCO unit produces a local concentration of hydroxyl radicals in the range typically produced by natural neutrophils in response to an infection. In some embodiments, the PCO unit produces a local concentration of hydroxyl radicals believed to be sufficient to oxidize a biofilm.

As stated above, it is believed that the PCO unit of the present invention may cause the production of hydrogen peroxide upon the semiconductor surface. Without wishing to be tied to a theory, it is believed that providing PCO upon an implant surface will produce singlet oxygen ($^1O_2$) through the following mechanism:

According to Allen, in the presence of sufficient halide, $H_2O_2$ is the rate limiting substrate for haloperoxidase microbicidal action. Microbicidal activity is linked to haloperoxidase generation of hypohalous acid:

$$\text{(Haloperoxidase)}$$
$$X^- + H_2O_2 \rightarrow HOX + H_2O \qquad (1),$$

and to the secondary generation of singlet molecular oxygen ($^1O_2$):

$$HOX + H_2O_2 \rightarrow {}^1O_2 + H_2O \qquad (2).$$

Both HOX and $^1O_2$ are antimicrobial reactants.

The present inventors have appreciated not only that PCO produces both superoxide ion and hydrogen peroxide, but also that typical human interstitial fluid contains a substantial amount of salts and so has significant amounts of $Cl^-$, a halide ion. Therefore, it is reasonable to conclude that the native halide ion present in the vicinity of the implant and the PCO-generated hydrogen peroxide may react to produce HOX, and this HOX will further react with another $H_2O_2$ molecule to produce singlet oxygen.*)*

It is well known that singlet oxygen is lethal to bacteria. In some embodiments, the PCO unit produces a local concentration of singlet oxygen believed to be sufficient to kill free-floating microbes. In some embodiments, the PCO unit produces a local concentration of singlet oxygen in the range typically produced by natural neutrophils in response to an infection. In some embodiments, the PCO unit produces a local concentration of singlet oxygen believed to be sufficient to oxidize a biofilm.

Although it appears that singlet oxygen is a very potent antibiotic, the extreme reactivity limits its sphere of influence. In particular, it is believed that singlet oxygen has an average lifetime on the order of milliseconds and a sphere of influence of only about 0.2 microns. Therefore, the production of singlet oxygen provides a comprehensive disinfecting response, but only very close to the surface of the implant- the nearby tissue is essentially unaffected.

Moreover, the present inventors have further appreciated the role played by chain reactions in ROS chemistry, and the need to insure that such reactions are self-limiting. Without wishing to be tied to a theory, it is believed that, since the production of singlet oxygen requires two hydrogen peroxide molecules, the above-stated reactions will be well-controlled due to the eventual depletion of hydrogen peroxide.

In addition, it has been recently reported by Wolfrum, ES&T, 2002, 36,3412-19 that photocatalytic oxidation effectively destroys biofilms. Wolfrum reported that the reactive oxygen species produced by its PCO unit effectively oxidized each of a phospholipid, a protein and a polysaccharide film. Since Wolfrum further stated that these substances were selected to be models of polymer-like biofilm, it is reasonable to conclude that PCO can not only destroy the biofilm protecting the foreign microbes, but in doing so it will expose the previously protected bacteria to lethal amounts of both hydroxyl radicals (OH) and superoxide radicals $O_2^-$.

Since it is known that the inner surface of a ventricular catheter is most prone to infection, in some embodiments, at least a portion of the inner surface of the catheter is coated with a photocatalytic layer. In some embodiments, at least half of the inner surface of the catheter is coated with a photocatalytic layer.

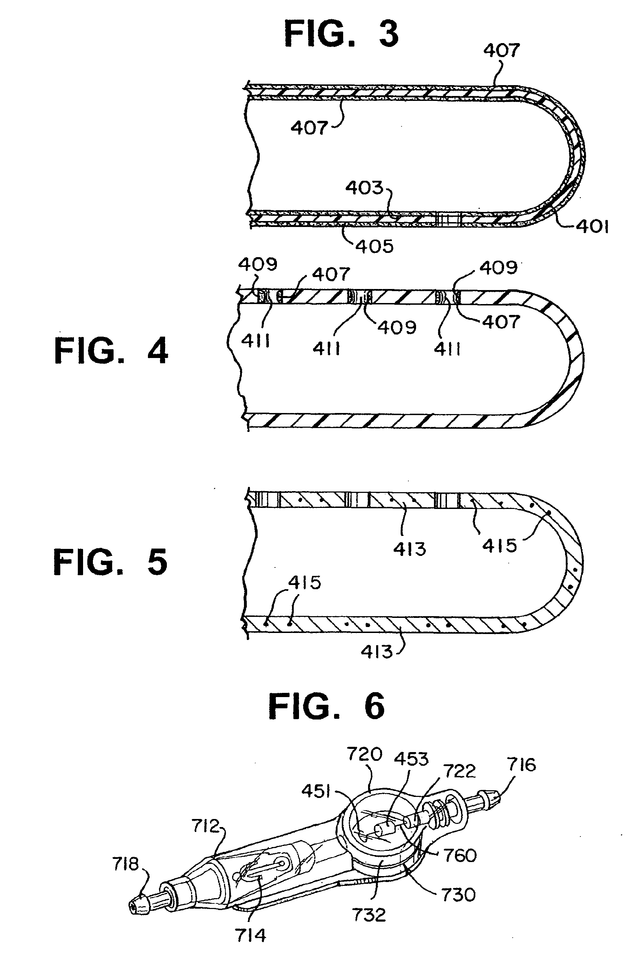

Now referring to FIG. 3, there is provided a cross-section of an end of a ventricular catheter component of a shunt of the present invention. In this embodiment, both the inner surface 403 and outer surface 405 of the silicone tube 401 are each coated with a layer of the photocatalytic material 407 (preferably, titania).

This embodiment is advantageous because the comprehensive coverage of the coating allows infection-fighting PCO to occur over essentially the entire catheter. In addition, the comprehensive coverage all protects essentially all of the silicone surface from any harmful oxidative effects of the inflammatory response to shunt insertion or the PCO reaction, thereby promoting the useful life of the silicone material.

Now referring to FIG. 4, there is provided a cross-section of an end of a ventricular catheter component of a shunt of the present invention. In this embodiment, the annular surfaces 409 of the silicone tube 401 defined by inlet holes 411 are each coated with a layer of the photocatalytic material 407 (preferably, titania).

This embodiment is advantageous because it is relatively easy to produce and yet allows PCO to take place in the vicinity of the inlet holes. Accordingly, this embodiment could be useful in situations in which the inlet hole becomes blocked.

Now referring to FIG. 5, there is provided a cross-section of an end of a ventricular catheter component of a shunt of the present invention. In this embodiment, the tube comprises a composite of a base material 413 and a photocatalytic material 415. In preferred embodiments, the base material comprises silicone (preferably, PDS) while the photocatalytic material comprises titania. In some embodiments, the photocatalytic material comprises between 0.10 vol% and 30 vol% of the composite.

This embodiment is advantageous because the unitary nature of the composite allows for its easy manufacturability, thereby avoiding the need for a coating step. Its unitary nature allows avoids the possibility of coating delamination. Lastly, PCO can be effected on essentially every surface of the tube.

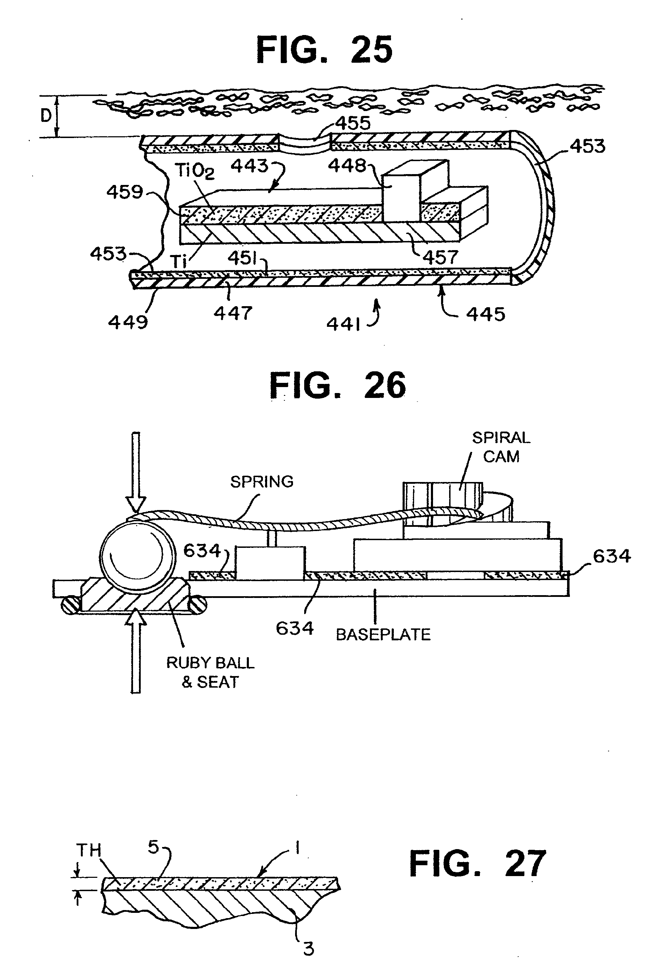

Now referring to FIG. 6, there is provided a schematic of a shunt of the present invention. In this embodiment, an Rf-receiving antenna 451 and a light emitting diode (LED) 453 are disposed between the shunt valve component 714 and the ventricular catheter inlet port 716. In preferred embodiments, the light emitting diode (LED) is AlGaN based and so emits UV light at a maximum wavelength of less than 380 nm, the shunt valve component is that shown in FIG. 26, and the ventricular catheter is a composite of PDS and titania.

In use, an external Rf transmitter transmits energy to the antenna. The antenna then powers the LED with an amount of energy needed to produce light. The light is then transferred into the ventricular catheter by fiber optic cable 760, where it excites the titania in the composite and produces PCO at the catheter surface. A battery or capacitor, timer/controller, along with a device for recharging the battery or capacitor, e.g., an antenna, piezoelectric device to convert mechanical motion/energy into electric energy may be used to power the LED.

This embodiment is advantageous because it allows the PCO reaction to be activated telemetrically, thereby obviating the need for invasive treatments due to blockage or infection.

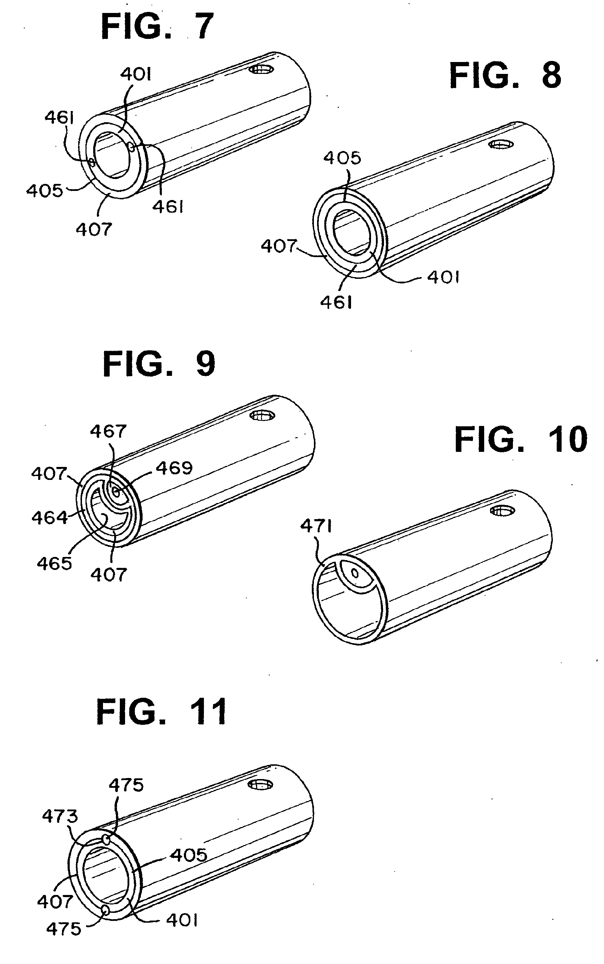

Now referring to FIG. 7, there is provided a cross-section of an end of a ventricular catheter component of a shunt of the present invention. In this embodiment, the outer surface 405 of the silicone tube 401 is coated with a layer of the photocatalytic material 407 (preferably, titania), and a plurality of longitudinal wave guides 461 are disposed within the silicon tube.

This embodiment is advantageous because the provision of a wave guide allows light to be more easily carried into the far end of the catheter, and because this design can be easily manufactured by simply co-extruding the wave guide along with the silicone tubing.

Now referring to FIG. 8, there is provided a cross-section of an end of a ventricular catheter component of a shunt of the present invention. In this embodiment, the outer surface 405 of the silicone tube 401 is coated with a wave guide material 461 , and the wave guide is coated with a layer of the photocatalytic material 407 (preferably, titania).

This embodiment is advantageous because the provision of a wave guide allows light to be more easily carried into the far end of the catheter, and because this design can be easily manufactured by applying simple dip coating technology.

Now referring to FIG. 9, there is provided a cross-section of an end of a ventricular catheter component of a shunt of the present invention. In this embodiment, tubing 464 comprises a CSF carrying lumen 465 and a fiber optic carrying lumen 467. In addition, both the inner surface and outer surface of the CSF carrying lumen are each coated with a layer of the photocatalytic material 407 (preferably, titania). Preferably, the tubing is made of silicone.In some embodiments, a fiber optic cable 469 is built into the shunt design during manufacture. In others, the cable can be inserted during therapy.

This embodiment is advantageous because it allows the shunt to carry CSF through lumen 467 to the valve and drainage catheter, and allows a fiber optic cable 469 inserted into the second lumen to provide local UV light to an area of the catheter in need of PCO.

Now referring to FIG. 10, there is provided a cross-section of an end of a ventricular catheter component of a shunt of the present invention. This embodiment is substantially similar to that of the dual lumen design of FIG. 9, except that the tubing material 471 comprises a composite of silicone and titania.

This embodiment is advantageous because it obviates the need to coat the lumen after manufacture of the tubing.

Now referring to FIG. 11, there is provided a cross-section of an end of a ventricular catheter component of a shunt of the present invention. In this embodiment, the outer surface 405 of the silicone tube 401 is coated with a layer of the photocatalytic material 407 (preferably, titania), and also comprises a plurality of longitudinal grooves 473 into which a longitudinal wave guide 475 are laid.

This embodiment is advantageous because the grooves can be easily made on the tubing surface either during tubing manufacture or after tubing manufacture, and the wave guide can then be easily laid into the grooves. In some embodiments, the grooves from at least a hemispherical surface to provide a snug fit. In some embodiments, the grooves can longitudinally form a helical pattern to enhance light distribution.

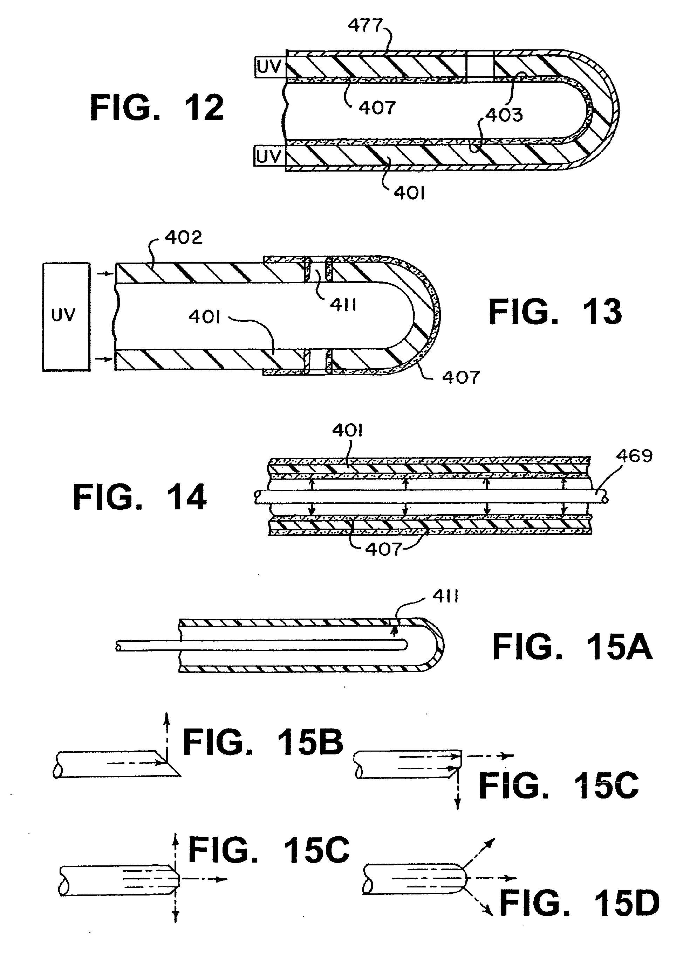

Now referring to FIG. 12, there is provided a cross-section of an end of a ventricular catheter component of a shunt of the present invention. In this embodiment, the inner surface 403 of the silicone tube 401 is coated with a layer of the photocatalytic material 407 (preferably, titania), while the outer surface 405 is coated with a layer of a refletive material 477 (preferably, silver).

This embodiment is advantageous because the reflective outer coating allows light to be transmitted and remain in the catheter. This feature is important because ventricular catheters are often oriented to have severe angle bends, and the reflective coating will provide the necessary bending of the light to allow its transmission into the further reaches of the catheter. In addition, the silver coating may also provide an anti-microbial effect.

In some embodiments (not shown), the reflective layer is designed to be only partially reflective and an additional photocatalytic layer is laid over it, thereby allowing for PCO to proceed on the outer surface of the catheter as well.

Now referring to FIG. 13, there is provided a schematic of light distribution into a cross-section a ventricular catheter component of a shunt of the present invention. In this embodiment, a light source disposed outside and proximal to the catheter emits light into an end portion 402 of the silicon tubing 401. Preferably, the catheter design is such that the light can travel through the tubing and reach the portion of the photocatalytic layer 407 surrounding inlet hole 411 in an amount effective to provide PCO.

This embodiment is advantageous because the light source can be conveniently located in either the valve area of the shunt (e.g, an LED) or light can be transmitted through a percutaneous needle to a known site in the valve area.

Now referring to FIG. 14, there is provided a schematic of light distribution into a cross-section a ventricular catheter component of a shunt of the present invention. In this embodiment, a fiber optic cable 469 is inserted into the lumen of the silicon tubing and and radially illuminates the length of the tubing. Preferably, the strength of illumination is such that an effective amount is transmitted through the tubing to the photocatalytic layer 407 residing on both the inner and the outer surfaces of the tubing.

This embodiment is advantageous because PCO activation can be made percutaneously by a fiber optic cable, thereby avoiding the need to manufacture complex light sources or light ports into the shunt design.

Now referring to FIGS. 15A-E, there is provided a schematic of light distribution into a cross-section a ventricular catheter component of a shunt of the present invention. In this embodiment, a fiber optic cable is inserted into the lumen of the silicon tubing and locally illuminates an area of the tubing surrounding an inlet hole 411. FIGS. 15B-E illustrate various shapes that the end of the fiber optic cable may comprise to distribute light in various desired directions.

A photocatalytic unit of the present invention comprises i) a light source and, ii) a photocatalytic surface comprising a semiconductor material to be irradiated by the light source. Without wishing to be tied to a theory, it is believed that, upon irradiation with an effective amount of UV light, the semiconductor material present in the photocatalytic surface produces holes and electrons. The holes catalyze the oxidation of water, thereby producing hydroxyl radicals OH. The electrons catalyze the reduction of oxygen, thereby producing superoxide radicals $O_2$-.

Preferably, the semiconductor material comprises a solid catalyst comprising a transition element, and more preferably is selected from the group consisting of titanium dioxide and ferric oxide. More preferably, it comprises titanium dioxide. In some embodiments, the semiconductor is Degussa P25, available from Degussa..

In some embodiments, the photocatalytic surface is produced by layering (preferably, by sonication) a powder comprising the semiconductor material upon a surface capable of being irradiated by the light source. In some preferred embodiments, an outer surface of a catheter is so layered with the photocatalytic material.

In some embodiments, since titania at least partially transmits UV light, the thickness of the oxidized layer may be sufficiently thick so as to also act as a waveguide. Therefore, in some embodiments, the photocatalytic surface has a thickness of between about 0.2 $\mu$m and about 10.0 $\mu$m, more preferably between about 2.0 and 4.0 $\mu$m.

In some embodiments, the photocatalytic surface is produced by providing sintered $TiO_2$ beads upon a shunt surface. In some embodiments thereof, the $TiO_2$ beads can create a porous scaffold. In this case, the porous scaffold comprising the semiconductor oxide provides two desirable qualities - disinfection capabilities (due to its photocatalytic qualities) and a convenient reaction zone for the photocatalytic process. The PCO unit can therefore be tuned to provide ROS throughout the reaction zone, while avoiding the diffusion of ROS outside the reaction zone.

In some embodiments, the photocatalytic surface is produced by using a sol-gel process to deposit titania upon a shunt surface.

In some embodiments, the photocatalytic surface is produced by using a vapor deposition process. Preferably, the vapor deposition process deposits titania upon a shunt surface (preferably, a catheter surface). In some embodiments, ion beam assisted sputtering methods are used to produce the photocatalytic surface.

Since photocatalysis is a surface phenomenon, the depth of the photocatalytic surface need not be particularly great. Moreover, it has been reported by Ohko, J. Biomed. Mat. Res. (Appl Biomat) 58: 97-101, 2001 that when $TiO_2$ thin films produced by heat treating exceed about 2 $\mu$m, the layer begins to peel from its substrate. Therefore, in some embodiments, the photocatalytic surface has a thickness of between about 0.2 $\mu$m and about 10.0 $\mu$m, more preferably between about 2.0 and 4.0 $\mu$m.

However, as discussed above, in some embodiments, the thickness of the photocatalytic layer may be so great as to act as a waveguide.

Preferably, the photocatalytic surface comprises a semiconductor material. More preferably, the semiconductor material is selected from the transition elements of the Periodic Table. More preferably, the semiconductor is selected from the group consisting of titanium dioxide and ferric oxide. More preferably, the semiconductor is titania. In some embodiments, the semiconductor is Degussa P25.

In some embodiments, the photocatalytic surface consists essentially of the semiconductor material. These embodiments have the advantage of manufacturing simplicity. In other embodiments, the photocatalytic surface can comprise a composite comprising at least a semiconductor material. Akin, J. Biomed. Mat. Res. 57, 588-596, 2001, discloses the preparation of macroporous titania films upon titanium surfaces. Akin's films were reported to be about 0.1 mm to about 1 mm in thickness. Pore sizes were reported to be 0.5 $\mu$m, 16 $\mu$m and 50 $\mu$m.

In some embodiments, the photocatalytic surface comprises a composite of a semiconductor material and a light-transmissible material. Preferably, the light transmissible material is a UV-transmissible material and is more preferably selected from the group consisting of alumina, sapphire and silica. This is advantageous in that this layer may also propagate light.

Moreover, when this composite is made into a porous scaffold, wherein the scaffold contains islands of $TiO_2$ interspersed throughout the porous scaffold. Because the UV light is not absorbed by the UV-transmissible portion of the material, the UV light is absorbed only by the titania interspersed throughout the scaffold. The titania present adjacent an internal scaffold surface then becomes photoactivated and produces ROS throughout the scaffold.

In some embodiments, the photocatalytic material may be compounded into the base material of the catheter to produce a composite catheter. The unitary nature of this embodiment eliminates the flaking issues typically associated with layered devices. In some composite-catheter embodiments, the base material of the catheter is PDS and the photocatalyst comprises titania. It is well known that titania is compatible with PDS. In this case, the PDS acts as a carrier for the titania and is UV-transmissive. Therefore, the physican need only illuminate the lumen in order to illuminate titania on both the inner and outer surfaces of the catheter composite.

In other embodiments, the composite comprises the base catheter material (such as silicone) and metal particles (such as titanium) coated with a photocatalytic layer (such as titania).

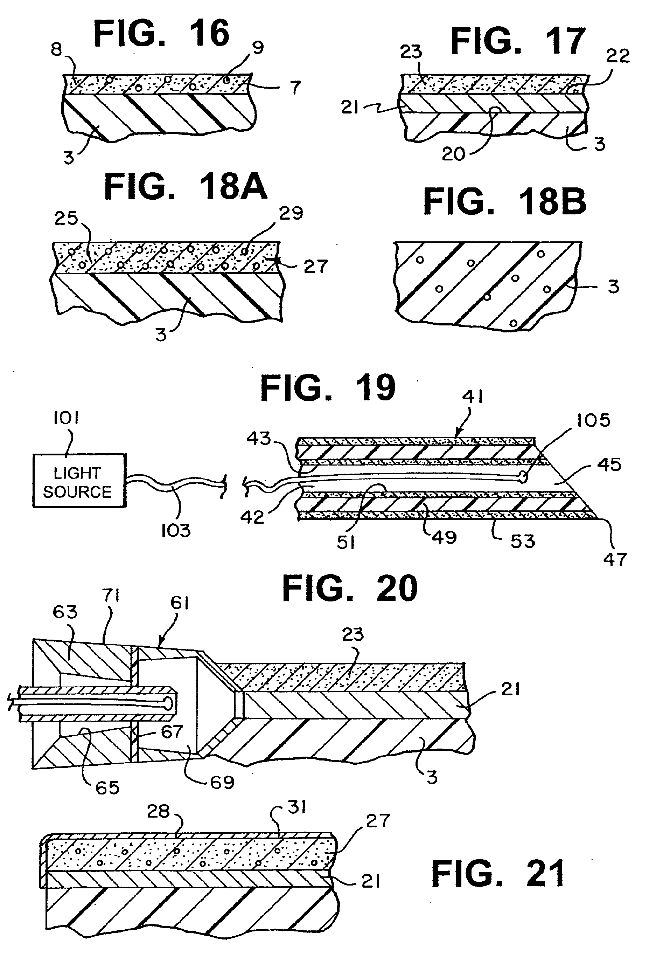

Now referring to FIG. 16, in some embodiments, the catheter comprises a base material 3 (such as silicone) overlain by a photocatalytic layer 7(such as titania). In this case, the photocatalytic layer 7 comprises a composite of a semiconductor material 8 doped with a dopant 9 that reduces the bandgap of the photocatalyst, thereby increasing the maximum wavelength of light absorbed by the photocatalytic layer. In some preferred embodiments, the dopant is selected from the group consisting of vanadium and chromium. It has been reported by Anpo et al, Pure Appl. Chem. Vol. 72, (7), 2000, pp. 1265-70 that when a dopant selected from this group is ion-implanted onto a titanium dioxide surface, the resulting surface is substantially photocatalytically active when irradiated with white light.

In some other preferred embodiments, the dopant is nitrogen. It has been reported by Lin, J. Mater. Chem., 2003,13(12)2996-3001 that when nitrogen is selected as the dopant, the resulting surface is substantially photocatalytically active when irradiated with light having either a 400 nm or a 550 nm wavelength.

In some other preferred embodiments, the dopant is selected from the group consisting of $Nd^{+3}$, $Pd^{+2}$, $Pt^{+4}$

and $Fe^{+3}$. It has been reported by Shah, PNAS, Ap. 30, 2002, 99(S.2), pp. 6482-6 that when one of these dopants is selected as the dopant, the resulting surface may be substantially photocatalytically active when irradiated with 450-460 nm light. Therefore, in some embodiments, the photocatalytic surface comprises a composite of a semiconductor material doped with a dopant that reduces the bandgap of the photocatalyst, thereby increasing the wavelength of light absorbed by the photocatalytic layer to include wavelengths greater than LTV.

In some preferred embodiments using a dopant, a titanium component of the shunt is oxidized to produce a titania surface layer, and this titania layer is then ion-bombarded with a dopant.

Since periprosthetic infections often form a biofilm that envelops a substantial portion of the surfaces of shunt catheters, it is appreciated by the present inventors that it would be highly desirable to photoirradiate substantially an entire inner and outer surfaces of the affected catheter. However, it is further appreciated that shunt catheters are typically quite long and made of materials having some capacity to absorb near UV light, and so are not conductive to complete irradiation from a single point light source. Moreover, the presence of light-absorbing brain tissue adjacent the photocatalytic surface further complicates the comprehensive irradiation of a surface of the catheter.

Now referring to FIG. 17, accordingly, in some embodiments, the catheter portion of the shunt further comprises a base material 3, a photocatalytic layer 23, and a waveguide 21 disposed in the base material and adapted to transmit light from a light source to distant surface portions of the catheter. Preferably, the waveguide comprises a material that is at least partially transmissible to UV or white light. When such a waveguide is provided within the base material (e.g., as a longitudinal fiber), the light irradiating the waveguide can travel via the waveguide throughout the surface of the photocatalytic layer. The advantage is that the light transmissible material acts as a wave guide, so that the UV light generated from the light source can spread laterally across the surface of the implant and thereby irradiate the photocatalytic layer from the back side.

In some embodiments, the catheter tubing is made of substantially clear silicone. Using clear silicone tubing may allow the tubing to also function as a waveguide to transmit light. However, since it is believed that silicone may absorb some portion of UV light, in some embodiments using clear silicone, the photocatalytic layers preferably comprise doped titanium dioxide and the desired photocatalysis is activated by a wavelength of visible light that is more readily transmitted by clear silicone.

In some embodiments, the wave guide 21 can be provided as a discrete layer between the inner surface 22 of the photocatalytic layer 23 and the outer surface 20 of the base material 3 of the catheter (as in FIG. 4). In such instances, the waveguide layer can be easily deposited by CVD processes.

Now referring to FIG. 18, in other embodiments, the wave guide can be provided as part of a composite layer 27 comprising the semiconductor material 29 and a light-transmissible (preferably, UV-transmissible) material 25. In this case, the composite layers acts as both a wave guide and a photocatalytic surface. In preferred embodiments, the composite comprises between about 0.10 vol% and 20 vol% semiconductor and between about 80 vol% and 90 vol% waveguide. In some preferred embodiments of the composite wave guide, composite is essentially dense (e.g., no more than 10 vol% porous), thereby providing strength.

In some embodiments, the light transmissible material is selected from the group consisting of a ceramic and a polymer. Suitable UV-transmissible ceramics include alumina, silica, $CaF_2$, titania and single crystal-sapphire. Suitable light transmissible polymers are preferably selected from the group consisting of polypropylene, polyesters, polycarbonate, silicone and acrylics. Because the catheters of the present invention should be highly flexible, it is preferably to use a polymer as the wave guide material of construction.

In some embodiments, a titanium wire is longitudinally embedded within at least one of the catheters. In this manner, the wire runs the length of the catheter and acts like a strip on a pinion. In this embodiment, the titanium wire can be used to both control the flexibility of the catheter as well as providing delivery of the therapeutic treatment.

Simple irradiation of any surface of the waveguide may be sufficient for the waveguide to propogate the light throughout the catheter and diffuse to the backside of the adjacent photocatalytic surface and generate ROS over that entire photocatalytic layer. Although comprehensive irradiation is easily accomplished when performed at the time of surgery (when the implant is visible to the surgeon), if ROS-based therapy is desired at some future, post-operative time, then the use of a minimally invasive fiber optic may be required to deliver the light, and so irradiation of the entire catheter surface may be more problematic.

Accordingly, and now referring to FIGS. 19 and 20, when a wave guide is used in conjunction with an external light source and light is usually transmitted to the wave guide via a fiber optic cable, it is desirable to provide a light port upon an inner portion of the wave guide in order to insure easy connection of the fiber optic to the wave guide.

FIG. 19 discloses a distal portion of a delivery needle 41 adapted to deliver the fiber optic to the waveguide. The needle 41 comprises a barrel 42 defining a small bore lumen 43 and a distal opening 45. The distal portion of the barrel forms a needle tip 47 suitable for penetrating an orthogonally-disposed seal and/or tissue (not shown). In some embodiments, the delivery needle can also be adapted to contain both a waveguide 49 and inner 51 and outer photocatalytic surfaces 53, so that the needle itself can be photo-sterilized and so not introduce bacteria into or draw bacteria from the implant site.

As shown in FIG. 19, the needle is adapted to house a fiber optic cable 103 that is connected to a light source 101. Light is generated in the light source, is transported through the fiber optic cable, and is emitted from the distal end 105 of the fiber optic cable.

Now referring to FIG.20, there is provided a hydrocephalus shunt comprising:

  a) a base catheter material 3,
  b) a waveguide 21 disposed within the base catheter material,
  c) a photocatalytic layer 23 overlying the base catheter material, and
  d) a light port 61 communicating with the waveguide and comprising:

  - a proximal receiving portion 63 adapted to receive and secure the delivery needle and comprising a throughbore 65,
  - an intermediate seal 67 sealing the throughbore, and
  - a distal barrel portion 69.

In FIG. 20, the proximal receiving portion of the light port comprises an inner bore 65 having a distally tapering circumference 71. It may also have a radio-opaque portion (not shown) that helps the surgeon find its location under fluoroscopy. The distally tapering circumference of the proximal receiving portion helps guide the needle into the proximal receiving portion. The proximal receiving portion may also have a securing means, such as a luer lock portion (not shown) in order to secure the needle within the light port. In some other embodiments, the securing means comprises a threaded recess adapted to mate with a threaded male distal portion of the delivery needle or fiber optic

The function of the intermediate seal 67 is to prevent tissue ingress to the light-communicating surface of the optically transmissible waveguide.

The function of the distal bore portion 69 is provide a space allowing for needle over-insertion, thereby minimizing physical damage to the waveguide portion of the implant.

If a wave guide is merely disposed within the catheter base material (or as an interlayer between an catheter base material and the semiconductor surface), then there is a possibility that light traveling within the wave guide will simply exit the far end of the wave guide and enter the adjacent tissue. In order to prevent such occurences and thereby enhance the efficiency of the light source, in some embodiments of the present invention, and now referring to FIG. 21, the catheter includes a reflective surface 31 adjacent an edge of the wave guide 21. The disposition of the reflective surface at an edge of the wave guide prevents laterally moving light from exiting the lateral edge of the wave guide, but rather reflects this light back into the wave guide and ultimately into the photocatalytic layer 7.

In other embodiments, the reflective coating is also placed on the outer surface 28 of a porous photocatalytic layer in order to reflect light escaping the photocatalytic layer back into the photocatalytic layer.

In preferred embodiments thereof, the reflective surface comprises a metal-containing layer, preferably coated upon a portion of the waveguide or photocatalytic surface. The metal-containing layer may be either a pure metal, a metal alloy, or even a metal oxide having a lower refractive index than the photocatalytic layer. In some embodiments, the metallic coating is selected from the group of metals consisting of silver and titanium. More preferably, silver is used in order to take advantage of its antimicrobial effect.

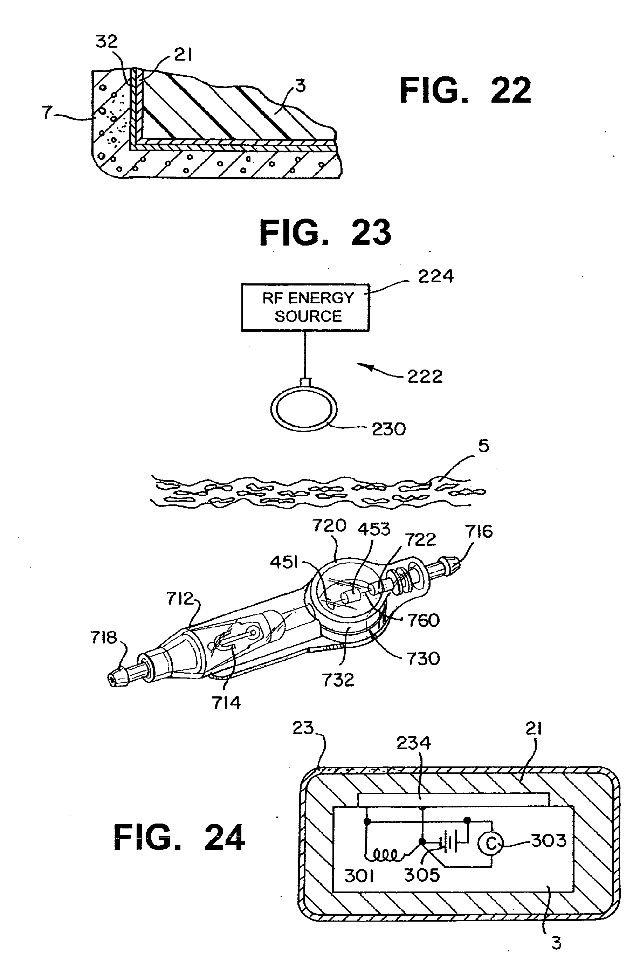

In some embodiments, the reflective surface comprises a multi-layer structure designed to create a reflection. For example, and now referring to FIG. 22, it may be desirable to use a multi-layer structure utilizing visible light; titania as the waveguide; and an external layer of vanadium-doped titania as the photocatalytic surface. In particular, FIG. 22 discloses an implant comprising:

  a) base catheter material 3,
  b) a wave guide layer (here, made of pure titania) 21 overlying the base catheter material,
  c) a partially reflective layer 32 (here, made of Ti, Ag, V or Cr) overlying the pure titania layer, and
  d) a white light-absorbing photocatalytic outer layer 7 (for example, a vanadium-doped titania layer).

When irradiated by white light, the waveguide layer does not generate any significant ROS (since the pure titania bandgap would be too high for light having a wavelength greater than 380 nm), but the external layer of vanadium-doped titania will generate the photocatalytic effect at or near the surface of the device in response to the white light, thereby providing ROS in the region of the infection.

In some embodiments (not shown), it is desirable to create a hole or window in the reflective layer to allow access by the fiber optic to the light port and to increase the light throughput to the waveguide. Because of this increased throughput, a thicker, more reflective layer (e.g., 80-90% reflective) can be suitably used with more efficiency.

Other light-related components, such as bifurcated fiber optic bundles and fluorescent or phosphorescent chemical mediators, that are designed to manipulate light and allow the light to reach remote surfaces of the device, are also contemplated by the present invention.

As noted above, the shunt of FIG. 1 is preferably treated by inserting a fiber optic cable into the lumen of the affected catheter. However, in some instances, the obstruction of the lumen may be so extensive so as to prevent distal access of the fiber optic cable. Accordingly, in some embodiments, a wave guide is effectively provided by selecting a dual lumen catheter as the catheter component. The dual lumen catheter comprises a first lumen in fluid communication with far end inlet holes and a second lumen having a closed far end. In use, the physician advances the fiber optic cable through the open end of the second lumen. Since this lumen is closed at its far end, it is protected from obstruction and so provides insured access of the fiber optic to the far end of the catheter.

In some embodiments, the light source is a UV light source. The UV light source is adapted to provide UV radiation to a UV-sensitive photocatalytic surface in an amount effective to produce a therapeutic amount of ROS. Preferably, the wavelength of the UV light is *UVA* light and emits light having a wavelength in the range of 320 and less than 380 nm. In this range, the UVA light effectively irradiates conventional $TiO_2$ and does not cause damage to DNA as does UVC light.

In some embodiments, the UV light source has a spectral maximum in the range of the UV and near-UV components of the solar spectrum. Preferably, the light source has a spectral maximum in the range of the near-UV components of the solar spectrum. Preferably, the light source has a spectral maximum in the range of less than about 380 nm, and is preferably between 300 nm and 380 nm. In some embodiments, the light source has a spectral maximum of about 365 nm.

Preferably, when UV or near UV light sources are selected, they are used in conjunction with semiconductor materials that exhibit photocatalytic activity when irradiated by UV or near UV light. One preferred semiconductor suitably used with UV light is titania.

In other embodiments, the light source is a white light source. The white light source is adapted to provide white light to the photocatalytic surface in an amount effective to reduce the local microbe concentration. Preferably, the wavelength of the white light is in the range of 380 nm- 780 nm. White light is particularly preferred because it effectively irradiates vanadium-doped $TiO_2$ or nitrogen-doped $TiO_2$ to produce photocatalysis and does not cause damage to DNA.

In some embodiments, using doped titania as the photocatalytic surface, visible light having a maximum absorption wavelength of between 400 nm and 650 nm is used. In some embodiments, using doped titania as the photocatalytic surface, visible light having a maximum absorption wavelength of between 450 nm and 600 nm is used. In some embodiments, using doped titania as the photocatalytic surface, visible light having a maximum absorption wavelength of between 450 nm and 500 nm is used.

The present inventors have appreciated that, in some situations, it may be possible to effectively irradiate an implanted shunt having a photocatalytic layer, wherein the irradiation is transcutaneous. It has been reported in the literature that the effective depth of penetration of light through the skin is wavelength dependent and is approximately as follows:

| Wavelength | Depth of Penetration |
| --- | --- |
| 380 nm | 1 mm |
| 600 nm | 4 mm |
| 780 nm | 10 mm |

Accordingly, if the selected photocatalytic layer becomes active when irradiated by, for example a 600 nm wavelength light, then that photocatalytic layer or a wave guide associated with the photocatalyst can be implanted at a depth of less than about 4 mm and transcutaneously irradiated to effectively produce the desired photocatalytic reaction.

In one embodiment, a shunt having a nitrogen-doped titania layer and an associated wave guide is implanted beneath the skin so that a near end of the wave guide is at a depth of about 3 mm. The near end of the wave guide is transcutaneously irradiated with 600 nm light. This light is then transmitted by the wave guide to the photocatalyst to produce a photocatalytic reaction in the shunt that provides the therapeutic ROS.

Since red light is easily transmitted through the skin to a depth of a few millimeters, it may be desirable to incorporate a light collecting surface on the top of the valve body that could be illuminated transdermally by a suitable light source, such as a diode laser or a low power HeNe laser. The transdermal delivery of light would be highly advantageous, in that it would eliminate the need for any invasion of the patient's skin in order to provide the desired therapy.

In order to divert this transdermally-delivered red light at a 90 degree angle from the light-collecting surface and propogate it towards the desired areas of the shunt, it may be useful to incorporate a number of angled shapes on the light-collecting surface. In some embodiment, those shapes may include pyramids, shingles or other raised shapes with an angled surface adapted to deflect the incident light at nearly a right angle (somewhat similar to a grating). These shapes could be molded into the housing, or made as a two-piece unit, and may be coated with a highly reflective layer, such as silver, to enhance deflection efficiency. The use of red light would necessitate the use of silver or other metal-doped titania for PCO activity.

In some embodiments, the light source is located external the patient. Providing an external light source simplifies the design of the photocatalytic shunt. In cases where irradiation occurs prior to the operation (to prevent infection) and the shunt is still outside the patient, the light source may be a light box. In cases where irradiation occurs during the operation and the patient's wound is open, the light source may be a conventional light source, such as a flood light or the operating room lights. In cases where irradiation or UV light with fiber optic wand to manually deliver light occurs after the operation and the patient's wound is closed, the light source preferably transmits light through a fiber optic cable having a proximal end connected to the light source and a distal end adapted for entry into the patient and connection to the shunt.

Preferably, the fiber optic cable used in conjunction with an external light source is adapted to have the strength and flexibility required to navigate within the catheter component of the shunt and the patient's tissues. This typically requires the cable to have a fine diameter. The proximal end of the fiber optic is adapted for connection to the external light source, while the distal end of the fiber optic is adapted for connection to a waveguide or lightport disposed upon the shunt. Activation of the light source sends light from the light source through the fiber optic cable and into the implant (preferably, the wave guide component of the implant).

Suitable fiber optic cable materials include quartz, plastic and silica, and are commonly available.

As shown above in FIG. 19, it is further preferable that a protective delivery needle 41 or catheter be used in conjunction with the fiber optic cable 103. The catheter has a long bore adapted to house the fiber optic and functions to protect the relatively thin fiber optic from undesired stresses encountered during navigation to the site of the shunt. The catheter can also serve as a protective shield that protects the surrounding tissue from any undesired effects caused by the light being transmitted through the fiber optic.

In order to insure against the spread of the infection by the catheter and/or fiber optic cable, each of these components may preferably be coated with a thin layer 51,53 of a photocatalytic material, such as titania. Irradiation of these thin layers by the light source can effectively sterilize each of these components. Further description of such a system is described in Ohko, supra.

In some embodiments, the light source is provided on the shunt itself and is adapted to be permanently implanted into the patient. The advantage of the internal light source is that, when a blockage or periprosthetic infection occurs post-operatively, there is no need for further transcutaneous invasion of the patient. Rather, the internally-disposed light source is activated by either a battery disposed on the shunt, or by telemetry, in order to produce ROS. In some embodiments of the present invention using an internal light source, the light source is provided by a bioMEMs component. In one embodiment thereof, the internal light source comprises a UV light source, and preferably comprises an AlGaN substrate. It has been reported by Stutzmann, Diamond and Related Materials, 11 (2002) 886-891, that AlGaN may have future application as a biosensor. Stutzman further conducted studies on the biocompatibility of GaN, AlGaN and AlN, and found very little interaction with living cell tissue, thereby suggesting the biocompatibility of these materials.

In some embodiments, the light source is situated to produce between about 0.1 watt and 100 watts of energy. Without wishing to be tied to a theory, it is believed that light transmission in this energy range will be sufficient to activate the photocatalytic surface on most shunt catheters. In some embodiments, the light source is situated to produce an energy intensity at the photocatalytic surface of between 0.1 watts/cm$^2$ and 10 watts/cm$^2$. In some embodiments, the light source is situated to produce about 1 milliwatt/cm$^2$. This latter value has been reported by Ohko et al., JBMR (Appl BioMat) 58: 97-101, 2001, to effectively irradiate a $TiO_2$ surface in an amount sufficient to produce a photocatalytic effect.

Since photocatalytic oxidation is generally believed to be a relatively ambient-temperature process, the heat produced by both the light source transmission and the desired oxidation reactions are believed to be negligible. That is, the temperature of the tissue surrounding the implant will not generally significantly increase during activation of the PCO unit, and so the surrounding tissue will not be thermally degraded by the therapies disclosed herein.

In some embodiments, there is provided a first exemplary PCO unit having an external light source. An externally based-control device has a light source for generating light within the shunt. The light generated by this source is transmitted through a fiber optic cable through the patient's skin to an internally-based waveguide light port provided on the shunt. The light port is adapted to be in light-communication with a wave guide disposed upon the outer surface surface of the shunt. A photocatalytic element disposed adjacent to the wave guide receives the light and produces photocatalysis.

Now referring to FIG. 23, there is provided a second exemplary PCO shunt having an internal light source. Externally based-control device 222 has an RF energy source 224 and an antenna 230 for transmitting signals to an internally-based antenna 451 provided on the shunt. These antennae may be electro-magnetically coupled to each other. The internal antenna 451 sends electrical power through a conductor overlying an insulator to a light emitting diode (LED) 453 disposed internally on the implant in response to the transmitted signal transmitted by the external antenna 230. The light generated by the LED travels across a wave guide and into the photocatalytic layer.

In some embodiments, the shunt further contains an internal power source, such as a battery or capacitor (not shown), which is controlled by an internal receiver and has sufficient energy stored therein to deliver electrical power to the light source of the PCO unit sufficient to cause the desired photocatalytic effect.

In some embodiments, the light generated by the internal PCO unit is powered by wireless telemetry integrated onto or into the prosthetic or implant itself. In the FIG. 23 embodiment, the receiver may comprise a radiofrequency-to-DC converter and modulator, wherein radiofrequency signals are emitted by the external antenna and picked up by the internal antenna. These signals are then converted by the receiver (not shown) into electrical current to activate the light source of the PCO unit.

In some embodiments, the telemetric portion of the shunt is provided by conventional, commercially-available components. For example, the externally-based power control device can be any conventional transmitter, preferably capable of transmitting at least about 40 milliwatts of energy to the internally-based antenna. Examples of such commercially available transmitters include Microstrain, Inc. Burlington, VT. Likewise, the internally-based power antenna can be any conventional antenna capable of producing at least about 40 milliwatts of energy in response to coupling with the externally-generated Rf signal. Examples of such commercially available antennae include those used in the Microstrain Strinlink™ device. Conventional transmitter-receiver telemetry is capable of transmitting up to about 500 milliwatts of energy to the internally-based antenna.

In some embodiments, and now referring to FIG. 24, the shunt includes a light emitting diode (LED) 234 built upon a base portion 3 of the shunt, along with the required components to achieve trans-dermal activation and powering of the device. These components can include, but are not limited to, RF coils 301, control circuitry 303, a battery 305, and a capacitor. Such a device could be capable of intermittent or sustained activation without penetrating the skin, thereby avoiding trauma to the patient and/or risk of infection from skin-borne bacteria.

As shown above, the accessory items needed to power and control the LED may be embedded within the shunt. However, they could also be located on the surface(s) of the shunt, or at a site adjacent to or near the shunt, and in communication with the shunt.

In some embodiments, the telemetry portion of the shunt is provided by vapor depositing a metallic material upon an appropriate insulating substrate, such as a titanium baseplate. For example, the internal antenna can be suitably manufactured by first creating an appropriate insulating substrate upon a baseplate surface and then CVD depositing a metallic layer in the form of a coil upon the insulating surface.

In some embodiments, the therapeutic ROS capabilities of the shunt of the present invention may be supplemented with an adjunct system for treating at least one of obstruction (catheter or valve), periprosthetic infection, and inflammation. One such system preferably comprises a pharmaceutical delivery system.

In some embodiments, the pharmaceutical delivery system is a coating comprising a pharmaceutical, wherein the coating is disposed upon a surface of the catheter. This coating acts as a sustained release device for the pharmaceutical that insures a constant introduction of the pharmaceutical into the surrounding tissue.

In some embodiments, the pharmaceutical delivery system comprises a drug pump containing a pharmaceutical. The drug pump can be activated either at the end of the surgery or afterward to provide a constant introduction of the pharmaceutical into the surrounding tissue.

In some embodiments, pharmaceutical delivery system comprises at least one channel created within or on catheter for delivering the pharmaceutical to a plurality of locations about the catheter surface. Preferably, the channel defines an entry port (preferably adapted for receiving a needle) located upon a first surface of the shunt and at least one exit port opening onto a surface of a catheter.

In preferred embodiments, the pharmaceutical is selected from the group consisting of an antibiotic, a growth factor and an anti-inflammatory.

Preferably, the antibiotic is delivered to the adjacent tissue in an amount effective to prevent a periprosthetic infection. Suitable antibiotics are desirably delivered in conventional prophylactic concentrations.

Preferably, the anti-inflammatory is delivered to the adjacent tissue in an amount effective to antagonize pro-inflammatory cytokines, and thereby prevent inflammation. The prevention of inflammation is believed to be particularly desirable because it is believed that inflammation (due to shunt implantation) is believed to be a prime cause of obstruction. Suitable anti-inflammatories include anti-TNF-$\alpha$ compounds and anti-interleukin-1$\beta$ compounds. Specific desirable compounds include (Remicade™).

On one preferred embodiment, the pharmaceutical delivery system comprises a silver halide coating. Without wishing to be tied to a theory, it is believed that the silver component of this coating becomes ionized following dissolution. Once ionized, it can entered the cellular membrane of adjacent cells and promote an intra-cellular reaction that produces singlet oxygen. It is believed that the singlet oxygen so produced has a lethal effect upon the invaded cell.

In some embodiments, hydrogen peroxide is delivered through the fluid delivery mechanism and is present in the vicinity of the photocatalytic layer. It has been reported in US Patent No. 4,861,484 ("Lichtin") that hydrogen peroxide has a significant synergistic effect upon the titania-based photocatalysis. For example, Lichtin reports that the destruction of certain organic compounds proceeds about 5-10 times as rapidly when titania is irradiated in the presence of hydrogen peroxide (as compared to its destruction rate when titania irradiated without hydrogen peroxide). Accordingly, it is believed that the provision of hydrogen peroxide with the present invention may enhance the effectiveness of the desired photocatalytic activity.

In some embodiments, a photosensitizer is delivered through the fluid delivery mechanism and is present in the vicinity of the photocatalytic layer. It has been reported by Wainright, J. Antimicrobial Chemotherapy, (1998) 42, 13-28, that local irradiation of photosensitizers (such as methylene blue) should be considered as a means for treating local infection due to their ability to produce singlet oxygen. Accordingly, it is believed that the additional provision of photosensitizers with the present invention may enhance the effectiveness of the desired photocatalytic activity.

In some embodiments, the photosensitizer is selected from the group consisting of phenothiazinium type, phenazine type, acridine type, cyanine type, porphyrin type, phthalocyanine type, psoralen type, and perylenequinonoid type.

In other embodiments, the photosensitizer is provided as a coating upon a shunt component (preferably a catheter surface).

In some embodiments, the catheter comprises a plurality of lumens wherein one of the secondary lumens has a distal outlet and is adapted to deliver protein-dissolving compounds, such as enzymes or peroxides, to the distal end of the catheter to the inlet holes. In some embodiments, the fluid may be delivered percutaneously, while in others it may be housed in an internal reservoir, preferably in conjunction with a suction or pumping mechanism to reduce extravasation of the material. Such fluid delivery could be manually activated. For example, in some embodiments, a fluid delivery system may comprise a bulb adapted to deliver treatment to the distal end of the catheter, and to provide suction to pull the dissolved material into the shunt. In some embodiments, the fluid delivery system is a MEMS-type system that is totally self-contained and provides sensor-based fluid delivery automatically. The sensor may be adapted to detect flow rate, predeterrmined chemicals or proteins, mass, or electrical resistance at the distal end and provide therapeutic delivery accordingly. Alternatively, fluid delivery could be based on a timer, wherein a prophylactic delivery of a small amount of the therapeutic fluid is delivered to the distal end of the catheter on a periodic, regular basis to maintain patency and prevent buildup.

In some embodiments, the shunt also includes mechanical means for providing therapy. In some embodiments thereof, the means may include a shaped-wire or Archimedes Screw disposed in a catheter lumen of the shunt. In use, the means may be actuated (e.g., by rotatation, vibration, or linear movement) to pump material through the shunt and also dislodge/disrupt protein or microbe adhesion to the distal end of the catheter. Actuation of the mechanical means could be selected from the group consisting of transdermal activation (e.g., by the patient with a bulb-type device), MEMS-based actuation, motor-based actuation (preferably including a rechargable battery), and transdermal energy transfer. In some embodiments, metal components used in the mechanical means may be silver or silver-coated for enhanced antimicrobial resistance. In other embodiments, metal components used in the mechanical means may be titanium and coated with titania for enhanced with PCO activity. In other embodiments, plastic components used in the mechanical means may be optically transmissive plastics adapted to transmit the light to the distal end of the catheter for PCO therapy.

Siloxane coatings are known to resist protein adhesion. Therefore, in some embodiments, at least one of the annular surfaces an inlet hole is coated with a siloxane coating (e.g., trimethyl chloromethylsilane).

In some instances, the shunt can be subject to therapeutic photocatalytic treatment prior to its implantation. Pre-implantation treatment is a preventative measure that can provide the surgeon with extra assurance that the shunt is sterile when it enters the body.

Providing a pre-implantation photocatalysis can also reduce the risk that transmissible diseases such as mad cow disease and AIDS become problematic.

In some pre-implantation embodiments thereof, the shunt can be placed in an aqueous slurry of titania particles and photoenergy can be applied to the slurry to produce the requisite photocatalysis. The ROS produced by the photocatalysis will oxidize not only any bacteria attached to the implant, but also problematic spores. It has been reported by Wolfrum, Environ. Sci. Tech., 2002, 36, 3412-19 that a titania-based reactor exposed to about 10 mW/cm$^2$ of 365 nm light is sufficient to kill *A. niger* spores.

Photocatalysis can also be provided upon the implant intra-operatively (i.e., during the surgery). For example, just prior to closing the patient, the surgeon can use a fiber optic to irradiate the photocatalyzable surface of the implant, thereby insuring that any bacteria that became attached to the implant during the surgery will be rendered ineffective. Without wishing to be tied to a theory, it is believed that a substantial percentage of problematic PPIs arise from infection occurring at the interface of the patient's bone and the implant.

It is further believed that intra-operative irradiation just prior to closing may be beneficial in reducing the extent of inflammation caused during the procedure.

Therefore, in some embodiments, the implant of the present invention is implanted into the patient and the PCO unit is then activated during the surgery. In some embodiments, the PCO unit activation occurs immediately prior to closing up the patient.

In some embodiments, the PCO unit activation occurs immediately after closing up the patient. For example, the patient is closed with the fiber optic still attached to the wave guide port. The surgeon then uses the fiber optic to irradiate the photocatalyzable surface of the implant, thereby insuring that any bacteria that became attached to the implant during the surgery will be rendered ineffective. After irradiation, the fiber optic is withdrawn from the patient.

For the purposes of the present invention, intra-operative and post-operative photocatalysis can each be considered to be a preventative treatment.

Since it is known that the valve components of a shunt are also susceptible to blockage or infection, it may also be desirable to provide PCO therapy to valve components. Therefore, in some embodiments, at least a portion of a valve component is coated with a photocatalytic layer. In some embodiments, the valve component to be coated is selected from the group consisting a baseplate, a ball, a seat, a spring and a stepper motor.

In some embodiments in which a ball or seat is coated with a photocatalytic layer, the ball or seat is made of a UV transparent material, such as silica or sapphire. In some embodiments, the seat is adapted to have a light port.

In some embodiments, the spring is made of titanium coated with titania.

Now referring to FIG. 25, there is provided a portion of a shunt 441 comprising a structural component 443 housed within tubing 445,
wherein the tubing comprises:

    a) a outer silicon tube 447 having an outer wall 449 and an inner wall 451,
    b) an inner photocatalytic layer 453 attached to the inner wall of the silicon tube, and
    c) a light port 455,

and wherein the structural component comprising:

    a) a baseplate 457 (in this case, made of titanium alloy) having an inner surface,
    b) a titania layer 459 disposed upon a first portion of the inner surface of the baseplate, and
    c) a valve component 448 disposed upon a second portion of the inner surface of the baseplate.

In practice, when the valved portion of the shunt becomes clogged (for example, by microbes that have formed a biofilm within the valved portion or by protein deposition), a cannula having a fiber optic cable (not shown) therein may be advanced through the skin and into the light port. Preferably, the distal end of the fiber optic cable is advanced to a location about half way between the inner titania layer of the tubing and the titania layer formed within the structural component. Upon activation of the UV light source, UV light will effectively irradiate both the titania layer of the tubing and the titania layer of the structural component, thereby causing the production of ROS. These ROS will then oxidize the biofilms, bacteria or proteinaceous matter within the portion to an extent effective to unclog the shunt.

Of note, if it is known that the hydrocephalus shunt can be situated within a depth D about 4 mm from the surface of the skin, and the photocatalytic layer may be made of a nitrogen-doped titania (so that it can be activated by ~600 nm light), then it may be possible to provide transcutaneous treatment of the device by irradiating the photocatalytic layer with 600 nm light. In this embodiment, a light collecting surface may be used to receive this 600 nm light just below the surface of the skin and distribute it within the shunt. As noted above, this 600 nm wavelength of light is believed to have a penetration depth of about 4 mm. Thus, in some embodiments, the shunt may be treated without having to remove the shunt or even breach the skin of the patient.

In some embodiments, hydrogen peroxide is also added to the clogged portion of the shunt, either through the light port or via an upstream fluid port. As discussed above, it is believed that the $H_2O_2$ will help accelerate the photocatalytic oxidiation reactions.

Now referring to FIG. 26, there is provided a photocatalytic layer 634 provided upon a titanium baseplate of a conventional hydrocephalus shunt.

In some embodiments, the PCO procedure effectively acts upon the target bacteria colony to eliminate at least 50% of the colony, more preferably at least 90%, more preferably at least 99%.

In some embodiments, the PCO procedure effectively essentially completely oxidizes the target bacteria to carbon dioxide and water.

In some embodiments, the ROS generated by the PCO unit are present in the reaction zone is an amount effective to disinfect the reaction zone. Typically bacteria that are considered to be prone to photocatalysis include, but are not limited to, staphylococcus epidermis. Microbes involved in mad cow disease and AIDS are also within the scope of the present invention. Staphylococcus epidermis is thought to be introduced into the patient through the normal flora of the skin. These types of bacteria tend to form a biofilm on the surface of the implant.

In some embodiments, the ROS generated by the PCO unit are present in the reaction zone in an amount effective to sterilize the reaction zone. The sterilization of the reaction zone means that spores in addition to bacteria are killed.

Now referring to FIG.27, in some embodiments, there is provided a shunt 1 of the present invention, wherein a photocatalytic layer 5 is produced by oxidizing a base material 3 comprising titanium, thereby producing a photocatalytic titania layer having a thickness TH of at least 0.2 $\mu$m.

An object is to provide a method of treating hydrocephalus, comprising the steps of:

a) inserting into a human cranium a hydrocephalus shunt having a component having a surface, and

b) producing reactive oxygen species on the component surface.

Preferably, the component surface comprises a photocatalytic material.

Further preferably, the component surface comprises a photocatalytic layer.

Preferably, the step of producing reactive oxygen species comprises illuminating the photocatalytic surface with light from a fiber optic cable.

Preferably, the step of producing reactive oxygen species comprises telemetrically illuminating the photocatalytic surface with light from a fiber optic cable.

Preferably, the component surface is a proximal catheter surface.

Preferably, the component surface is a distal catheter surface.

Preferably, the component surface is a valve component surface

Preferably, the ROS are produced in an amount sufficient to kill microbes present in the vicinity of the shunt.

Preferably, the ROS are produced in an amount sufficient to oxidize organic mater present within a lumen of the shunt.

Preferably, the ROS are produced in an amount sufficient to reduce inflammation in the vicinity of the shunt.

Preferably, the component surface comprises a silver coating.

Preferably, the component surface comprises a photosensitizer coating.

Preferably, the step of producing ROS comprises the step of applying a voltage through the component surface.

It is another objective to provide a shunt having a catheter comprising a photocatalytic material.

Preferably, the photocatalytic material comprises a semiconductor oxide, more preferably a titanium dioxide selected from the group consisting of anatase and rutile, and mixtures thereof.

Preferably, the photocatalytic material comprises a dopant.

Preferably, the photocatalytic material is present upon an inside surface of the catheter.

Preferably, the photocatalytic material is present upon an outside surface of the catheter.

Preferably, the catheter comprises an inside surface, an outside surface and an inlet hole providing fluid connection therebetween, and wherein the photocatalytic material is present upon a surface of the inlet hole.

Preferably, the catheter is made of a composite material comprising the photocatalytic material, more preferably the composite comprises poly(dimethylsiloxane), even more preferably, the photocatalytic material comprises titania. Preferably, the photocatalytic material comprises between 0.1 vol% and 30 vol% of the composite.

Preferably, the catheter comprises poly(dimethylsiloxane).

It is yet another objective to provide a hydrocephalus shunt having a catheter comprising a wave guide.

Preferably, the wave guide comprises at least one fiber, which preferably comprises glass or a polymer such as a polymer selected from the group consisting of silicones, urethanes, acrylics and polycarbonates.

Preferably, the catheter comprises a first lumen adapted for transported CSF, and a second lumen adapted for carrying the wave guide. Preferably, the second lumen has a fiber optic cable contained therein.

Preferably, the shunt further comprises a light port adapted for transmitting light to the wave guide.

Preferably, the wave guide has a transmissivity to _ nm (UV) light of at least 90%.

Preferably, the catheter is a composite and the wave guide is present as a component of the composite, which preferably comprises a glass oxide or a polymer.

Preferably, the shunt further comprises an LED adapted for transmitting light to the wave guide.

A yet further objective is to provide a method of manufacturing a hydrocephalus shunt having a valve component and a catheter component having a photocatalytic material, comprising the step of:

attaching the catheter component to the valve component.

Preferably, the catheter comprises a base material having a surface, and the photocatalytic material is coated upon the surface. Preferably, the coating is a sol-gel coating. Preferably, the coating is applied by sonication.

An even yet further objective is to provide a hydrocephalus shunt comprising a catheter having a surface having a silver coating thereon.

Preferably, the silver coating is present upon an inside surface of the catheter.

Preferably, the silver coating is present upon an outside surface of the catheter.

Preferably, the catheter comprises an inside surface, an outside surface and an inlet hole providing fluid connection therebetween, and wherein the silver coating is present upon a surface of the inlet hole.

Preferably, the catheter is made of a composite material comprising silver.

Preferably, the composite comprises poly(dimethylsiloxane).

Yet another objective is to provide a hydrocephalus shunt comprising a catheter comprising a memory metal.

Preferably, the memory metal is present upon an outer surface of the catheter.

Preferably, the memory metal comprises nickel and titanium.

Preferably, a portion of the memory metal an oxidized crystalline layer.

**Claims**

1. A hydrocephalus shunt comprising a light source.

2. The shunt of claim 1 wherein the light source is an LED.

3. The shunt of claim 1 or 2 wherein the light source is adapted to transmit UV light.

4. The shunt of any of claims 1 to 3 wherein the light source comprises AlGaN.

5. The shunt of any of claims 1 to 4 wherein the light source is battery operated.

6. The shunt of any of claims 1 to 4 further comprising an antenna, wherein the light source is powered by the antenna.

7. The shunt of any of claims 1 to 6 further comprising a catheter, and wherein the light source is adapted to transmit light to the catheter.

8. The shunt of claim 7 wherein the catheter further comprises a wave guide, and wherein the light source is adapted to transmit light to the wave guide.

9. The shunt of any of claims 1 to 6 further comprising a proximal catheter and a distal catheter, and wherein the light source is located between the catheters.

10. The shunt of claim 9 further comprising a housing comprising a valve, wherein the housing is located between the catheters.

11. The shunt of claim 10 wherein the housing contains the light source.

12. The shunt of claim 11 wherein the light source is outside the housing, preferably proximal to the housing.

13. A hydrocephalus shunt comprising a light port.

14. The shunt of claim 13 further comprising a proximal catheter and a distal catheter, and wherein the light port is located between the catheters.

15. The shunt of claim 14 further comprising a housing comprising a valve, wherein the housing is located between the catheters.

16. The shunt of claim 15 wherein the housing contains the light port.

17. The shunt of claim 15 wherein the light port is outside the housing, preferably proximal to the housing.

FIG. 1A

FIG. 1B

FIG. 2B

FIG. 2A

EP 1 961 442 A1

# FIG. 3

# FIG. 4

# FIG. 5

# FIG. 6

# FIG. 7

# FIG. 8

# FIG. 9

# FIG. 10

# FIG. 11

**FIG. 12**

477
407
403
UV
UV
401

**FIG. 13**

402
UV
401
411
407

**FIG. 14**

401
469
407

**FIG. 15A**

411

**FIG. 15B**

**FIG. 15C**

**FIG. 15C**

**FIG. 15D**

# FIG. 16

# FIG. 17

# FIG. 18A

# FIG. 18B

# FIG. 19

LIGHT
SOURCE

# FIG. 20

# FIG. 21

# FIG. 22

# FIG. 23

RF ENERGY
SOURCE

# FIG. 24

## FIG. 25

## FIG. 26

SPIRAL
CAM

SPRING

634

634

RUBY BALL
& SEAT

634

BASEPLATE

## FIG. 27

**European Patent Office**

## EUROPEAN SEARCH REPORT

Application Number

EP 08 00 9084

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 93/21973 A (UNIV LOMA LINDA MED [US]) 11 November 1993 (1993-11-11) | 13,14 | INV. A61M27/00 |
| A | * page 14, lines 12-31 * | 1-12, 15-17 | |
| | * page 16, lines 5-8 * ----- | | |
| X | US 5 957 912 A (HEITZMANN HAROLD A [US]) 28 September 1999 (1999-09-28) | 13 | |
| A | * column 5, line 45 - line 49; figures 10,13 * ----- | 1-12, 14-17 | |
| A | WO 96/28200 A (MEDTRONIC PS MEDICAL [US]) 19 September 1996 (1996-09-19) * page 9, paragraph 5; figure 2 * ----- | 1-17 | |
| A | EP 0 888 795 A (JOHNSON & JOHNSON PROFESSIONAL [US]) 7 January 1999 (1999-01-07) * the whole document * ----- | 1-17 | |
| A | US 4 950 232 A (RUZICKA PETR [US] ET AL) 21 August 1990 (1990-08-21) * column 4, line 63 - line 68 * ----- | 1-17 | TECHNICAL FIELDS SEARCHED (IPC) A61M |
| A | JP 2003 260126 A (KANAGAWA KAGAKU GIJUTSU AKAD; JAPAN SCIENCE & TECH CORP) 16 September 2003 (2003-09-16) * abstract * ----- | 1-17 | |

The present search report has been drawn up for all claims

| Place of search | Date of completion of the search | Examiner |
|---|---|---|
| Munich | 9 July 2008 | Cuiper, Ralf |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 08 00 9084

This annex lists the patent family members relating to the patent documents cited in the above-mentioned European search report.
The members are as contained in the European Patent Office EDP file on
The European Patent Office is in no way liable for these particulars which are merely given for the purpose of information.

09-07-2008

| Patent document cited in search report | | Publication date | Patent family member(s) | | Publication date |
|---|---|---|---|---|---|
| WO 9321973 | A | 11-11-1993 | AT | 160945 T | 15-12-1997 |
| | | | AU | 4104193 A | 29-11-1993 |
| | | | CN | 1081622 A | 09-02-1994 |
| | | | DE | 69315688 D1 | 22-01-1998 |
| | | | EP | 0637971 A1 | 15-02-1995 |
| | | | US | 5385541 A | 31-01-1995 |
| US 5957912 | A | 28-09-1999 | AU | 3648299 A | 01-11-1999 |
| | | | WO | 9952585 A1 | 21-10-1999 |
| WO 9628200 | A | 19-09-1996 | AU | 5312496 A | 02-10-1996 |
| | | | US | 5738666 A | 14-04-1998 |
| EP 0888795 | A | 07-01-1999 | DE | 69808558 D1 | 14-11-2002 |
| | | | DE | 69808558 T2 | 26-06-2003 |
| | | | ES | 2185114 T3 | 16-04-2003 |
| | | | JP | 11146911 A | 02-06-1999 |
| | | | US | 5928182 A | 27-07-1999 |
| US 4950232 | A | 21-08-1990 | NONE | | |
| JP 2003260126 | A | 16-09-2003 | NONE | | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 3886948 A
- US 4332255 A
- US 4387715 A
- US 4551128 A
- US 4595390 A
- US 4615691 A
- US 4772257 A
- US 5928182 A
- US 4816016 A
- US 5176627 A
- US 10774105 B
- US 10459406 B
- US 20040254522 A
- US 4861484 A

**Non-patent literature cited in the description**

- **SHANBHAG.** *J. Biomed. Mar. Res.,* 1992, vol. 26, 185-95
- **KEREIAKES.** *Circulation,* 2003, vol. 108, 1310-5
- **CHOU.** Mononuclear cell inflammatory infiltrate in conjunction with monocyte colony stimulating factor has been implicated in the pathogenesis of vascular smooth muscle cell apoptosis, depletion and subsequent weakening of plaque infrastructural integrity, which precipitates plaque rupture. *Catheterization and Cardiovascular Interventions,* 2002, vol. 57, 387-94
- **KAPLAN et al.** *J. Biomed. Mat. Res.,* 1992, vol. 26, 1039-51
- **WOLFRUM.** *ES&T,* 2002, vol. 36, 3412-19
- **OHKO.** *J. Biomed. Mat. Res. (Appl Biomat),* 2001, vol. 58, 97-101
- **AKIN.** *J. Biomed. Mat. Res.,* 2001, vol. 57, 588-596
- **ANPO et al.** *Pure Appl. Chem.,* 2000, vol. 72 (7), 1265-70
- **LIN.** *J. Mater. Chem.,* 2003, vol. 13 (12), 2996-3001
- **SHAH.** *PNAS,* 30 April 2002, vol. 99 (S.2), 6482-6
- **STUTZMANN.** *Diamond and Related Materials,* 2002, vol. 11, 886-891
- **OHKO et al.** *JBMR (Appl BioMat),* 2001, vol. 58, 97-101
- **WAINRIGHT.** *J. Antimicrobial Chemotherapy,* 1998, vol. 42, 13-28
- **WOLFRUM.** *Environ. Sci. Tech.,* 2002, vol. 36, 3412-19